# EUROPEAN PATENT APPLICATION

(11) **EP 1 772 112 A2**
(43) Date of publication of application: **11.04.2007**
(21) Application number: 06021144.8
(22) Date of filing: 09.10.2006
(51) Int. Cl.: A61B 19/02, A61M 5/32, G06F 17/00

(54) **Remote monitoring of medical device**

(30) Priority: 07.10.2005 US 246901
(71) Applicant: Sherwood Services AG, 8200 Schaffhausen (CH)
(72) Inventor: Japuntich, John C., Harvard, IL 60033 (US)
(74) Representative: Tomlinson, Edward James

(57) **Abstract**

The present application is directed to a remotely monitored medical device. A sensor (24) associated with the device (22) detects its state and generates a signal representative of the sensed state. Based on the sensor signal, a remote notification device receives information indicative of the device's state according to a messaging protocol. The notification device informs a user of the sensed state so that appropriate action is taken.

## Description

### BACKGROUND

Hospitals and other healthcare facilities often use hypodermic needles, lances, and other items capable of puncturing or cutting into human skin. After being used, such items, collectively known as "sharps", pose a risk of contamination and injury by accidental puncture and should be disposed of very carefully. Special waste disposal containers have been designed to confine the sharps and reduce the risk of accidental puncture. Accepted practices call for these containers to be collected, removed from the healthcare environment, and disposed of, perhaps in several stages. In some instances, a service organization separate from the healthcare facility carries out the final container disposal stage. The collected containers are then replaced with empty containers to repeat the cycle.

A variety of sharps containers are known in the art. For example, U.S. Patent No. 5,947,285 to Gaba et al., discloses a medical waste disposal system for contaminated products. This system has a hollow disposal container and a housing enclosure that is engageable with and covers the container. The enclosure of Gaba et al. has an opening and a tumbler that is pivotally mounted to prevent access to the interior of the enclosure when a contaminated product is being deposited into the interior of the disposal container.

In U.S. Patent No. 5,387,735 to Ponsi et al., a disposal container is provided together with a disposal system employing the disposal container. The container of Ponsi et al. comprises a hollow container body that has an opening at the top to permit access to the interior of the container body and a barrier disposed adjacent the opening for restricting access to the interior of the container body. An outer enclosure of the Ponsi et al. system is shaped to accommodate the inner container.

U.S. Patent Nos. D478,663 and D478,664, and U.S. Patent Application Publication No. 20030213714, disclose additional container systems. Also, exemplary container systems, such as disclosed in Panck, Jr. et al., U.S. Patent No. 6,712,207, are available from Tyco Healthcare Group LP, Mansfield, Massachusetts.

Though optional, such container systems generally include a receptacle sized to receive sharps and other medical waste for disposal, a cover assembly configured to permit a user to deposit sharps in the receptacle while restricting the user's access to the interior of the receptacle, and an outer enclosure configured to at least partially receive the receptacle and cover assembly for mounting to a wall while restricting unauthorized removal of the receptacle and cover assembly.

If a container becomes full before being collected and, thus, cannot accept another sharp, that sharp may go unconfined for a period of time in which it poses a hazard. Conversely, a container collected well before becoming full presents an inefficient use of the container's capacity and an unnecessary added expense to the facility.

Conventional sharps disposal containers sometimes provide alarms or indicators when they become full. For example, U.S. Patent No. 5,918,739 to Bilof et al., shows an emitter and detector mounted on a container so that the emitter projects a beam across the interior of the container at a predetermined level toward the detector. Once the level of sharps in the container reaches the predetermined level and continuously breaks the beam, an indicator or alarm provides an indication that the container has become full. Bilof et al. also disclose actuating an indicator each time an object passes through the beam for counting the number of sharps deposited in the container. Nonetheless, such conventional level indicators still require personal inspections and fairly vigilant monitoring of containers.

### SUMMARY OF THE INVENTION

Embodiments of the invention overcome one or more deficiencies in known systems by permitting remote monitoring of a medical device, such as a medical waste container. A message configured or otherwise formatted according to a messaging protocol contains information representative of a sensed state of the monitored device. By communicating the sensed state information to a remote location, aspects of the invention permit a user to be notified so that appropriate action is taken.

A medical waste container monitoring system embodying aspects of the invention includes a sensor associated with a medical waste container. The sensor senses a state of the waste container and generates a signal representative of the sensed state. A remotely located notification device receives a message that contains information representative of the sensed state according to a messaging protocol. The notification device is responsive to the message for providing a notification to the user.

According to another aspect of the invention, a medical device includes a medical waste container configured to receive one or more medical waste items deposited in it. A sensor associated with the waste container senses a state of the waste container and generates a signal representative of the sensed state. The device also includes a transmitter responsive to the sensor signal for transmitting a message to a receiver located remotely from the transmitter. The message is transmitted according to a messaging protocol and contains information representative of the sensed state of the waste container.

In accordance with further aspects of the invention, a method of monitoring medical waste containers includes sensing a state of a medical waste container and communicating a message containing information representative of the sensed state of the waste container. The message is configured according to a messaging protocol and transported using a communications protocol. The method also includes remotely notifying a user of the sensed state of the waste container in response to the communicated information.

In yet another aspect of the present invention, a data structure includes at least a header, a message type, and a data section, which define a message configured according to a messaging protocol for communication using a selected communications protocol. The header permits messaging according to the selected communications protocol, the data section contains information representative of a sensed state of a medical device, and the message type defines an operation to be performed on the data section.

Alternatively, embodiments of the invention may comprise various other methods and apparatuses.

Other features will be in part apparent and in part pointed out hereinafter.

### BRIEF DESCRIPTION OF THE DRAWINGS

FIG. 1 is a block diagram illustrating components of a medical device monitoring system embodying aspects of the invention.

FIG. 2 is a block diagram illustrating components of a medical device monitoring system embodying further aspects of the invention

FIG. 3 is a block diagram illustrating components of a medical device monitoring system embodying further aspects of the invention.

FIG. 4 is a block diagram of a sensor for use in the system of FIGS. 1, 2, or 3.

FIG. 5 is a block diagram of exemplary computer-executable components of the sensor of FIG. 4.

FIG. 6 is a block diagram illustrating components of an exemplary network environment including a medical waste container monitoring system embodying aspects of the invention.

FIGS. 7A-7D are exemplary flow diagrams illustrating notification processes according to aspects of the invention.

FIG. 8 is a block diagram illustrating components of an exemplary messaging protocol embodying aspects of the invention.

FIG. 9 is a block diagram illustrating components of a medical waste container monitoring system embodying yet further aspects of the invention.

FIG. 10 is a block diagram illustrating components of a medical waste container monitoring system embodying yet further aspects of the invention.

FIG. 11 is an exemplary flow diagram illustrating operation of a medical waste container monitoring system embodying aspects of the invention.

Corresponding reference characters indicate corresponding parts throughout the drawings.

### DETAILED DESCRIPTION

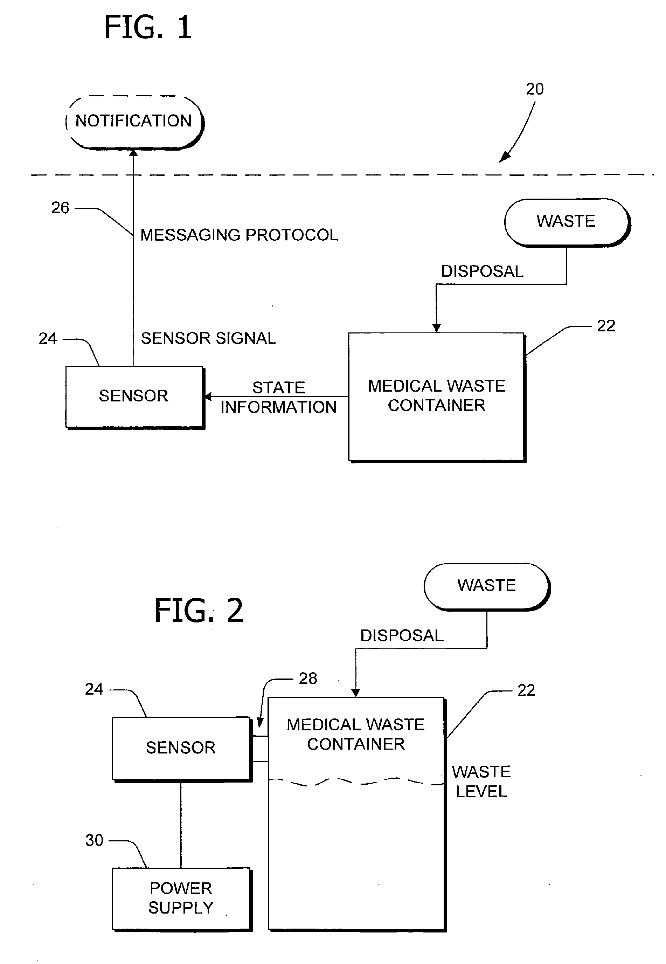

Referring now to the drawings, FIG. 1 illustrates components of a medical waste container monitoring system 20. Aspects of the invention permit a user to be notified remotely about a condition or state of medical device, such as a medical waste container 22. Such state information is, for example, an indication of whether the waste container 22 is full or empty, or the waste container 22 is scheduled for replacement. In one embodiment, waste container 22 is an approved sharps disposal container into which used hypodermic needles, lances, and the like is deposited.

Although described in the context of sharps disposal, aspects of the invention are applied to other medical devices including any disposal container for various forms of medical waste generated in the diagnosis, treatment, or immunization of human beings or animals or in medical-related research, production, and testing. Such waste includes, but is not limited to: needles; lancets; blood-soaked bandages; culture dishes and other glassware; discarded surgical gloves; discarded surgical instruments; cultures, stocks, and swabs used to innoculate cultures; and removed body organs and tissue. A list of suitable waste containers for use in embodiments of the present invention is found in Tyco Healthcare's Sharp Disposal catalog number H-46935. Also, the waste container 22 may be mobile. An example of a mobile waste container and a horizontal drop lid container is found in the aforementioned catalog. For instance, the container is releaseably secured to a cart found in the aforementioned catalog.

The container 22 is disposable (filled once or a limited number of times and then disposed) or reusable (filled, emptied, and reused). It is also fashioned from a material that is at least partially transmissive to one or more types of emission, such as light or sound, to enable some methods of detecting the amount of sharps or other waste items in the container.

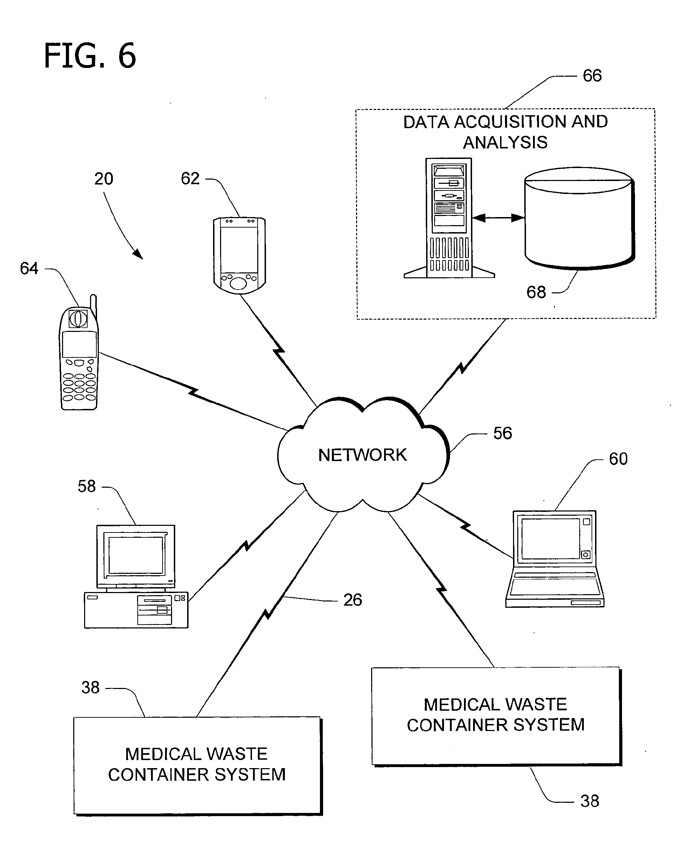

As shown in FIG. 1, a sensor 24 senses the state of waste container 22 and generates a signal representative of the sensed state. In response to the sensor signal, a user receives a notification. According to embodiments of the invention, the system 20 communicates the notification using a messaging protocol 26 over a network (e.g., network 56 of FIG. 6) to the user at a location remote from waste container 22. The user (or data acquisition system 66 of FIG. 6) acts on the transmitted state of the waste container 22. The act is a response acknowledging the state, an email to a service provider or a message to alarm, by way of a blinking LED, at the waste container to warn medical providers not to introduce further material into the container 22. The state of waste container 22 sensed by sensor 24 is representative of one or more of the following: a level of medical waste items deposited in container 22 relative to its capacity; an uneven stacking condition within container 22, a count of medical waste items deposited in container 22, a date or time of last servicing of the waste container 22, and a service history of container 22.

In one embodiment, sensor 24 is positioned in proximity to container 22 and is configured to detect a level of sharps in the container or another characteristic such as those described above. Numerous sensors and sensor systems are usable, examples of which are disclosed below. The sensor 24 is mounted directly on or near waste container 22 or on a housing that encloses the container. Also, in FIG. 2, the sensor 24 is disposable or reusable. The sensor 24 is releaseably secured using means such as hinges 28, bolts, screws, tape or a strap into or onto the medical waste container 22. This allows the medical practitioner or service provider to adjust the height of sensor 24 to increase the frequency of waste removal in the event of, for example, a more hazardous waste item material.

As is well known in the art, a power source or supply 30 provides power for various components, such as sensor 24. The power source 30 is located anywhere with respect to container 22. Moreover, the power source 30 is attached to an alternating current (not shown) or use batteries (not shown). The power source 30 is also a solar cell array. A wall cabinet or enclosure, perhaps mounted on a wall, defines an interior size to receive at least a portion of waste container 22. The wall cabinet also has a reusable or temporary waste container 22. Examples are found in the aforementioned catalog.

In operation, as waste items are deposited in container 22, the sensor 24 detects a level, for example, within the container's interior cavity and generates a sensor signal indicating the detected fill level. The sensor 24 generates a signal representative of a condition in a continuous broadcast or intermittently.

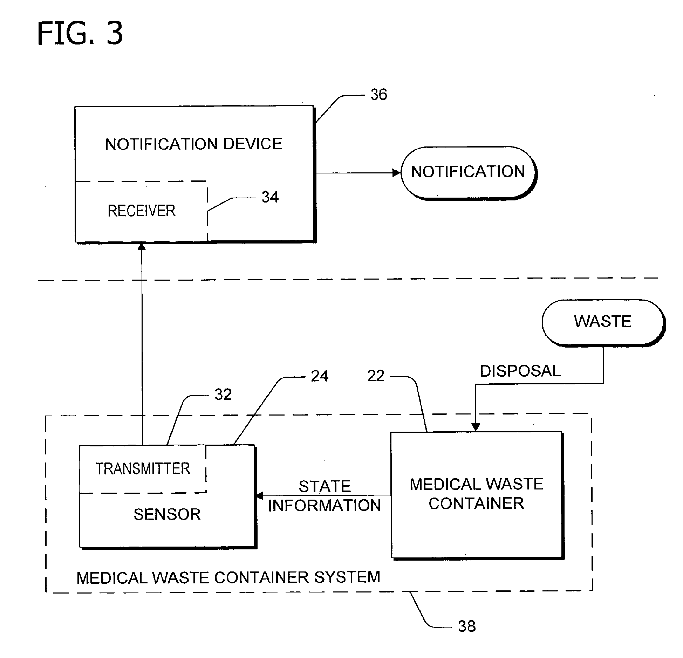

Referring now to FIG. 3, a transmitter 32 or other such input/output device is responsive to the signal from sensor 24 for transmitting information pertaining to the sensed state of container 22. The transmitter 32, which is for example embodied by a transceiver, is configured to receive the signal from sensor 24 continuously, intermittently, following an interrogation signal to sensor 24 from transmitter 32, or in some combination thereof or alternative interval. In one embodiment, transmitter 32 communicates with a remotely located receiver 34 according using the messaging protocol 26 over a communications protocol. A notification device 36, which is associated with the receiver 34, provides a notification representative of the sensed state to a user. For example, the notification informs the user when waste container 22 is full and in need of emptying or replacement. In this manner, aspects of the invention permit remote monitoring of container 22. As shown in FIG. 2, waste container 22, sensor 24, and transmitter 32 comprise an exemplary medical waste container system 38.

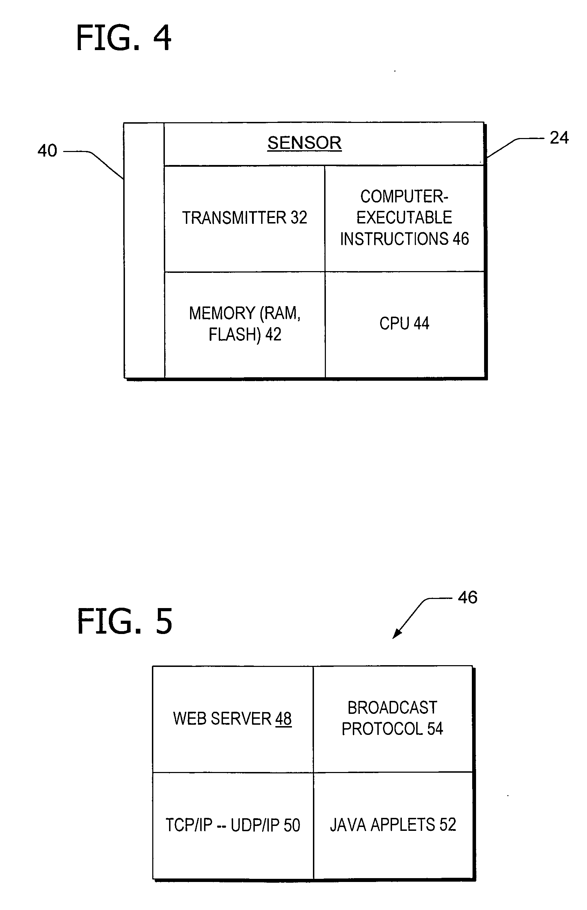

Referring to FIG. 4, sensor 24 has one or more edge connectors 40 to plug and play various input and output devices (e.g., a personal digital assistant 62, cell phone 64, or cabling from a computer 58 as shown in FIG. 6), and the edge connector 40 is interfaceable to a weight or volume device as discussed below. Plug and play sensors are known in the art. For example, such sensors have embedded memory chips holding technical data that describe the particular sensor's specific characteristics. The information is stored in what is known as a Transducer Electronic Data Sheet (TEDS). The sensor 24 in one embodiment includes the transmitter 32 as well as a memory 42 and a microprocessor 44. The memory 42 has random access memory, flash, or a releaseably removable memory stick such as those available from I-O Data of Kanazawa, Japan or Micron Technology, Inc. of Boise, Idaho. The memory 42 holds computer instructions 46, sensor readings, and/or a response to a sensor notification.

As shown in FIG. 5, computer instructions 46 are the embedded protocols as discussed below, a web server application 48, TCP/IP or UDP/IP 50 communications protocol, java applets 52, and/or a broadcast protocol 54 such as real time publisher and subscriber protocols operating using the TCP/IP or UDP/IP stacks. Those skilled in the art are familiar with the use of the TCP/UDP/IP protocol suite as the underlying framework upon which Ethernet communications are built. Because these transport-level protocols are too low level to be used directly by any but the simplest applications, higher-level protocols such as HTTP, FTP, DHCP, DCE, RTP, DCOM, and CORBA are used. Modifying the publish-subscribe protocol (Real-Time Publish-Subscribe), for example, adds parameters to offer application developers an easy way to manage communication on bus with different deadline requirements. Distributed application developers have several choices for writing communications, including known communication architectures such as Point-to-Point, Client-Server, and Publish-Subscribe.

In operation, sensor 24 starts or boots up after the power supply 30 is turned on. The sensor 24 goes through a start-up procedure verifying memory 42, checking transmitter 32, loading saved settings from memory 42 (e.g., flash), and determining if other I/O devices such as a memory stick are connected. If sensor 24 is a node on a network, the CPU 44 executes an address resolution protocol instruction set, for example, to register itself with the network manager, which is located on the remote computing devices 58, 60, or 66 in FIG. 6.

After boot up or restart, sensor 24 is ready for operation. As discussed above, the general transmission of information is request / reply or publisher / subscriber. Depending upon the configuration stored in memory 42, the system boots in the publisher / subscriber mode. The sensor 24 continuously broadcasts on UPD/IP a message for a specific period of time. As described above, notification device 36 messages the user, request an input acknowledgement and then respond to the broadcast message. The reply is stored in memory 42 located in the sensor 24, memory in the notification device 36 or in a database 68 (see FIG. 6).

Alternatively, the user requests data from the sensor by querying a particular internet protocol address over the network 56 using a PDA 62, cell phone 64 or remoter computer 58. The sensor 24 is suitable to have computer instructions 46 representative of a web server 48. The PDA 48, for instance, requests sensor 24 data and the server 48 replys with the information to the user interface (not shown) in the PDA 48.

As described above, sensor 24 and/or transmitter 32 and notification device 36 communicate via a communication link according to a communications standard using a messaging protocol 26. The communication link is in the form of any electronic wired or wireless communication system, such as a two-way radio, wireless telephony system, etc. It is appreciated that the communications link utilizes any one of a variety of communications media and/or communication protocols or methods to transfer data. Examples of suitable wire communications media/methods include, but are not limited to, public switch telephone networks ("PSTN"), wired digital data networks, such as the internet or a local area network ("LAN"), coaxial cable, fiber optic cable and the like. Examples of suitable wireless communications media/methods include, but are not limited to, wireless telephony including analog cellular, digital personal communications service ("PCS"), short message service ("SMS"), and wireless application protocol ("WAP"). Other suitable wireless communication media/methods include wireless digital data networks, such as IEEE 802.11 wireless LAN ("WLAN"), two-way paging networks, specialized mobile radio systems, infrared, and non-licensed ISM-service communication links, such as Bluetooth.

A web page is suitable to have applets 52 or additional computer instructions located in the page for further processing in the notification device 36. The applets 52 prompts the user (not shown) to respond, save the transmitted data or cause further processing of the data such as storing in a database 68 or transmitting to a data acquisition system 66.

Further, some communication methods, either wired or wireless, include serial (RS-232 or RS-485) or Ethernet which use CAN or Internet protocol ("IP") addressing, CAN (Controller Area Network), which is a serial bus system comprising the data link layer of the seven layer ISO/OSI reference model, generally provides two communication services, namely, the sending of a message (data frame transmission) and the requesting of a message (remote transmission request, RTR). As is known in the art, CAN is based on a broadcast communication mechanism, which is based on a message-oriented transmission protocol. It defines message contents rather than stations and station addresses. Every message has a message identifier that is unique within the whole network because it defines content and also the priority of the message. At this time, the CAN protocol supports two message frame formats. A "CAN base frame" supports a length of 11 bits for the identifier (formerly known as CAN 2.0 A), and a "CAN extended frame" supports a length of 29 bits for the identifier (formerly known as CAN 2.0 B). One skilled in the relevant art will appreciate that additional or alternative communication media/methods are practiced and are considered within the scope of the present invention. Accordingly, those of ordinary skill in the art will appreciate that the communications link is constructed with commercially available circuitry necessary for the particular type of communication described herein. For example, in one embodiment, the communication link is constructed from components analogous to the electronics used for a two-way radio system commonly used in a home alarm system. These features are described in Saltzstein et al., U.S. Patent No. 6,727,814, which describes obtaining status information from a portable medical device and communicating this information to a remote system or user.

Communication between the various components of the present invention and, for example, notification of the need to attend to a waste container 22 is implemented using a hospital monitoring network and a resource management system. A hospital monitoring network is a communications network such as an Ethernet network supporting transmission control protocol/internet protocol (TCP/IP) standards or any other applicable communication protocols. In one embodiment, hospital monitoring network is optionally built using industry-standard equipment, for example IEEE 802.3 cabling. A plurality of electronic devices and more particularly hospital devices such as, but not limited to, computers, patient monitors, patient meters, and patient sensors, and other communication and interfacing devices. These devices are for example nodes on communications network 56 and capable of messaging 26 state information about the waste container 22.

In this regard, the communications protocol employed by monitoring system 20 is a wireless communications protocol, an internet protocol or a protocol that uses an internet protocol (including FTP, TCP/IP, and Ethernet/IP), a serial line communications protocol, a Bluetooth link protocol, a field bus protocol such as PROFibus, Modbus, and Modbus/TCP, a controller area network protocol such as CANOpen, Ethernet-based protocols such as PROFinet, MODBUS/TCP and the like.

In an alternative embodiment, the sensor signal also activates a local alert. For example, this signal activates a level indicator or provides some other form of notification to a user in the vicinity of waste container 22. The indicator is an analog display, digital display, a source of light or a source of sound, or any other known indicia communicable to users proximal to the indicator. For example, the indicator provides a visual indication of container fill level through a lighting system (red -full, yellow - 3/4 full, and green 1/2 full or less), a "gas gauge" fill level indicator, or the like. A similar indicator scheme issuitabke to be employed at the remote location.

Thus, monitoring system 20 is used to determine, for example, essentially immediately when container 22 is full, or nearly full. This permits a user to promptly remove waste container 22 and replace it with an empty one. In addition, the sensor signal is useable to activate indicia such as a light or sound to locally alert a user, including one in the immediate vicinity of container 22, of the sensed condition. Such signals from sensors are optionally communicated to a central point in order to facilitate container collection and disposal.

In one embodiment, notification device 36 comprises a node on a data communications network 56 (e.g., the internet) as shown in FIG. 6. For example, a personal computer 58 located remotely from waste container 22 provides a notification of the state of container 22 via its display. The sensor 22 communicates with the computer 58 directly via the network 56 or indirectly via transmitter 32.

In the embodiment of FIG. 6, medical waste container system 36 transmits the sensed state of container 22 (e.g., level of each in-room sharps container) to computer 58 via wireless transmission, as disclosed above. The computer 58 serves as a central computer from which nurses on the floor (or anyone else who would have access and a need to know the level of waste containers 22) monitor the container states in a designated area. Using network 56, additional computing devices or communications devices 60, 62, 64 have access to the same data available on computer 58. For example, computer 58 provides notifications that permit a service provider to regularly monitor the status of a number of containers 22. This allows the service provider to determine which hospitals or other container sites to visit and which containers to exchange for empty containers.

Similarly, computer 58 or another one of the devices 60, 62, 64 allow, for example, a hospital's environmental services or custodial personnel, depending on who changes out containers 22, to regularly monitor the status information to determine which rooms to visit on a given day and which containers 22 to change out.

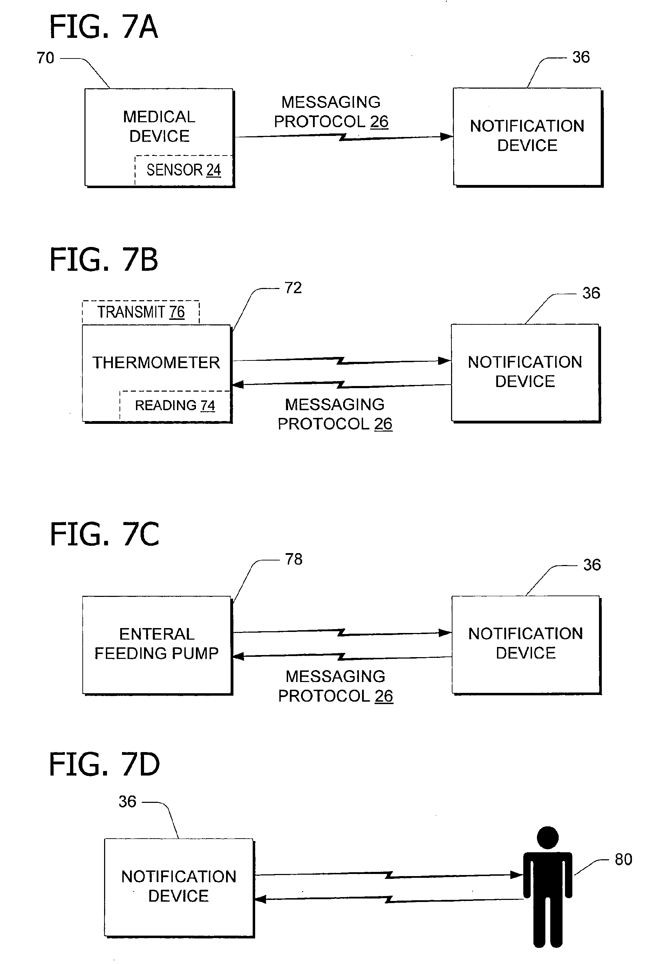

As described above, waste container 22, sensor 24, and transmitter 32 comprise an exemplary medical waste container system 38. Alternatively, as shown in FIG. 7A, any medical device 70 capable of measuring is suitable to be integrated to a sensor 24 for transmitting and receiving messages. For example, medical device 70 is a medical waste container, a thermometer, an enteral feeding pump, a compression apparatus for venuous treatment, or the like.

In the embodiment of FIG. 7B, a thermometer 72 transmits its temperature reading 74 or a series of temperature readings to notification device 36 or to the database 68 of data acquisition system 66. The user transmits the temperature reading 74 using a transmit button 76, or computer instructions (not shown) embedded in the thermometer 72. In the alternative, thermometer 72 broadcasts (as described above) when the temperature reading is complete. Applicable thermometer devices are the Fas-Temp manufactured by Tyco Healthcare Group, LP and U.S. Patent No 6,634,789 for an Electronic Thermometer, a Tympanic thermometer disclosed in U.S. Patent No. 6,238,089, U.S. Patent No. 5,857,775 for a Thermometer Probe having a Watertight Seal, or pending application WO US2003/011606 Tympanic Thermometer Nozzle Design filed April 15, 2003.

FIG. 7C illustrates a similar exemplary embodiment in which the medical device is an enteral feeding pump 78. The general construction and operation of the enteral feeding pump 78 is generally the same as disclosed in co-assigned U.S. Patent Application Nos. 10/853,958 filed May 24, 2004 and entitled Administration Feeding Set And Valve Mechanism, 10/854,136 filed May 24, 2004 and entitled Flow Control Apparatus, and 10/853,926 filed May 25, 2004 entitled Flow Monitoring System For A Flow Control Apparatus.

U.S. Patent No. 6,610,021, discloses a suitable compression apparatus (not shown) for use in connection with embodiments of the present invention. For example, a pair of compression sleeves and a manifold tubing set is provided as an integral unit. The tubing set comprises a first set of conduits integrally connected to the first sleeve and a second set of conduits integrally connected to the second sleeve. The first and second conduit sets are connected at a controller connection device comprising a manifold, having a plurality of fluidly isolated, branched fluid passages, and a connector for plugging directly into a controller for a source of compressed air. The only connection that must be made prior to operation of the device is the connection at the controller.

FIG. 7D diagrammatically illustrates the delivery of a notification to a user 80 and a response.

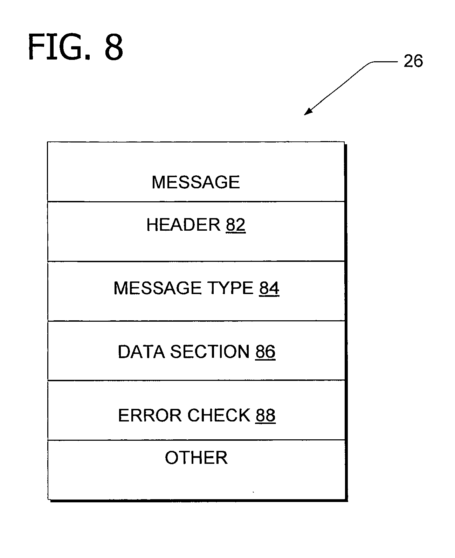

Referring to FIG. 8, in one embodiment the message format according to messaging protocol 26 has a header 82, message type 84, data section 86, and error checking section 88. The notification device 36, which is for example a personal digital assistant 62, cell phone 64 or remote computer 58, is the subscriber in this embodiment. The subscriber decodes the message to read the data and acts on the information. A typical message format is MODBUS/TCP, which has a header, function code and data set. As is known in the art, MODBUS is an application-layer messaging protocol, positioned at level 7 of the OSI model. It provides client/server communication between devices connected on different types of buses or networks. The header 82 allows messaging over a selected protocol such as TCP/IP. The function code or message type 84 defines the operation such as read or write with sensor 24. The data section 86, which includes the sensed state is the information read or written from or to sensor 24 into or out of the notification device 36. The messaging protocol 26 sends a specific function code or message type 84 having a defined data 86 for receipt at the notification device 36. Depending on the header 82, the notification device 36 reads and processes the message or ignores it. The message protocol 26 in this example is bi-directional, both reading and writing between the notification device 36 and medical device 70. Alternatively, the medical device is an enteral feeding pump such as the peristaltic pumps sold by Tyco Healthcare Group, LP and described in US Pat. No. 5,584,671, Apparatus for Delivering Fluid to a Patient and pending applications Flow Monitoring System for a Flow Control Apparatus, US Serial Number 10/480,428 filed 25-May-2004; Flow Control Apparatus, US Serial Number 10/854,136 filed 25-May-2004; and Re-Certification System for a Flow Control Apparatus US Serial Number 10/945,758 filed 25-May-2004. The above patents and patent applications are currently owned by Tyco Healthcare.

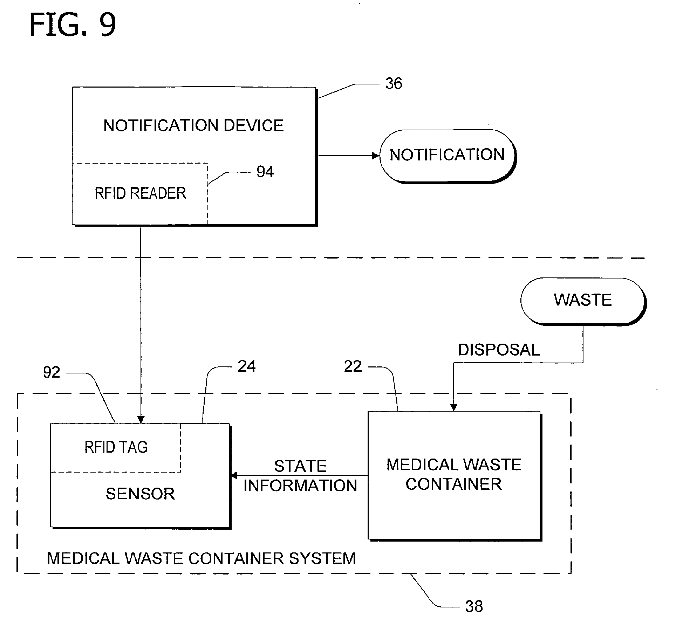

FIG. 9 illustrates yet another embodiment of the invention in which monitoring system 20 utilizes a radio frequency identification (RFID) tag 92 responsive to the sensor signal for storing information representative of the sensed state of waste container 22. In this embodiment, an RFID reader 94 associated with notification device 36 sends an interrogation signal to the RFID tag 92 for retrieving the stored information. The notification device 36 in turn is responsive to the information read by the RFID reader 94 to provide the notification to the user. The RFID tag 92 is passive and, thus, powered by the reader 94. Those skilled in the art are familiar with four common ISM (industrial, scientific, medical) frequency band in which RFID communications occur: 128 kHz, 13.56 MHz, 915 MHz, and 2.45 GHz. Standards used for RFID tagging include ISO 14443 and EPCglobal, among others. The RFID tag 92 is read by a reader located on the hospital floor. A typical reader is sold by Sensormatic a division of Tyco International LTD.

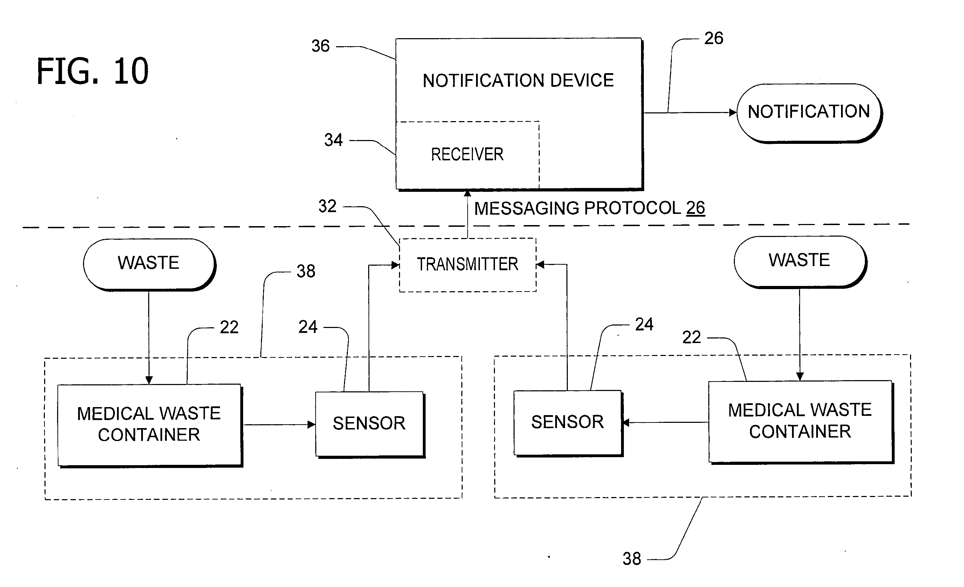

In the embodiment of FIG. 10, transmitter 32 is responsive to sensor signals monitoring a plurality of medical waste container systems 38.

Further to the examples in the context of sharps disposal, transmitter 32 transmits information concerning the sharps (e.g., level, quantity, weight, etc.) to computer 58, which is located remotely from container 22 and which receives information from other sources as well. Information then flows from computer 58 via network 56 to other devices and computers such as shown in FIG. 6 that are located within one or more organizations (e.g., environmental services in a healthcare facility or a sharps disposal service external to the facility). In this manner, users are then alerted to empty or remove a filled container 22 and replace it with an empty one.

Depending on the user, the notification indicate, "Service -- Time to visit Hospital XX and change out sharps containers in rooms a, b, c, & d before they register FULL" or "Hospital Staff -- Time to change out sharps containers in rooms a, b, c, & d before they register FULL."

In one embodiment, the monitoring system 20 also includes a data analysis computer 66 for monitoring and analyzing information representative of conditions present at one or more waste containers 22. A database 68 associated with the computer 66 stores, for example, usage information per each monitored container location gathered from the sensor signals. In this manner, the data analysis computer 66 permits a user to optimize container usage efficiency and save money by changing out only those containers 22 that need to be changed. Users seek to avoid changing out a partially filled container 22 simply because service is due on the rotation. Likewise, users seek to avoid delay in changing out a full or over-filled container 22 because this presents a possible health hazard. Usage tracking and analysis also permits detection of usage anomalies. For example, if a particular sharps container location has a normal usage rate of one 3-gallon container every four days and usage spikes to one 3-gallon container every day, the relatively dramatic change in usage is suitable indicate an anomaly. In this example, a user finds that non-sharps waste (e.g., cups, gloves, paper, etc.) is being deposited in the container. In yet another embodiment, data analysis permits inventory management and/or an automated ordering system for ordering replacement containers 22. It is to be understood that computer 58 may also comprise the data analysis computer.

In an alternative embodiment, a managed use server or computer system is optionally coupled to and in communication with a hospital monitoring network. A managed use system further includes a modem coupled thereto for communications across a standard telephone line or other communication lines such as coaxial lines, fiberoptic lines, cellular, radio or satellite signal lines. The managed use system is preferably used to communicate with a hospital equipment provider, or a service provider for a hospital equipment supplier. The managed use system collects information from the hospital monitoring network communicates that information via modem to a service provider who processes the information and sends out a bill, based on the information collected, to the hospital. In this managed use embodiment and others, the data gathered regarding the state of a medical device are suitable for automatic order entry to signal the manufacturer/distributor that a sharps container, for example, has been changed / replaced and to place an order for a replacement unit to be made / shipped to the user. In the alternative, other consumables associated with the medical device being monitored are also suitable to be automatically ordered in response to the data.

A work order system database is optionally coupled to the hospital enterprise network. In one embodiment, work order system is WOSYST.RTM. for Windows.RTM. available from St. Croix Systems, Inc., St. Croix Falls, Wis. The work order system is configured to schedule maintenance based on length of ownership of devices such as telemetry tower, patient monitor, configured monitor, network interface device, Octanet, monitors or sensors, and any other devices that may be coupled to the network.

These and other details of hospital monitoring networks and resource management systems are contained in Gary, Jr. et al., U.S. Patent No. 6,640,246, and McMenimen et al., U.S. Patent Application Publication No. 20030061123.

As described above, the communications protocol employed by monitoring system 20 is a wireless communications protocol, an internet protocol or a protocol that uses an internet protocol (including FTP, TCP/IP, and Ethernet/IP), a serial line communications protocol, a field bus protocol such as PROFibus, or Modbus, an area network protocol such as CANOpen, Ethernet-based protocols such as PROFinet, Ethernet IP and ModBus/TCP and the like. Technologies for communicating among transmitter 32 (and RFID tag 92) and receiver 34 (and RFID reader 94) and computer 58 (and other devices 60, 62, 64) are classified according to the communication medium and the signal type or message format 26. Transmitter or media types include the broad category of wireless, RFID, voice over IP, infrared, broadband, serial, power line, and acoustic modem, among others. Signal types include numerous protocols or communication standards such as those mentioned above.

Brackett et al., U.S. Patent No. 6,826,578, discloses an infrastructure for collecting and distributing clinical data for data mining. In this example, clinical data that has been stored as a structured reporting (SR) object (e.g., DICOM SR, HL-7 and XML) is collected for use in data mining. In the context of clinical data, Brackett et al. utilize the emerging DICOM SR standard along with other SR standards thereby removing the need for data re-entry. The clinical data that is collected for data mining is already stored in a defined and known format within each hospital. This hospital specific clinical data is then transformed into a common format and stored in a data repository for use in data mining. An embodiment of the present invention also establishes an infrastructure for the collection of these standard SR objects. In addition, the clinical data records, or reports, are collected without duplication and without workflow inhibition. An embodiment also establishes an infrastructure for distributing the SR objects, or providing data mining on the SR objects, using a web-based interface for creating user defined searches. In addition, bundles of medical reports are provided to companies to do their own data mining research.

Referring now to sensor 24 in greater detail, aspects of the invention are not limited to a single type of sensor. For example, a sensor system for determining the level of sharps in container 22 are implemented using numerous technologies. Among the many technology possibilities are those based on: optics, weight measurement, volume measurement, density measurement, ultrasonics, RF capacitance, inductance, microwaves, acoustic or electronic resonance frequency shifts, imaging, radioactivity, proximity, solid sensors, contact sensors, and subsurface sensors.

Variations within each of these technologies are utilized. Optical sensors, for example, are optionally based on infrared or visible light detection. They may also make use of a tape which changes opacity. In place of light, other types of emissions are used, including microwaves, radiowaves and other electromagnetic waves, sound waves (ultrasonics), particles (such as electrons) or radioactive emissions. With any form of emission and detection, both reflection and transmission are exploited in sensor system construction.

Weight sensors are utilized as well. Weight sensors are optionally constructed based on springs or strain gauges, the latter being in turn based on a piezoelectric crystal or film.

Imaging systems optionally use video cameras or image detectors such as charged-coupled devices (CCD), CMOS imagers, or particle detectors. Radiographic imaging is also employed using a detector of radioactivity and a source of radioactivity, either imposed on the system or emanating directly from the contents of container 22.

Any of these technologies and others are used to count the number of sharps deposited in container 22, determine a level of sharps relative to the container's capacity, rate of sharps disposal, manner of disposal from this count, or the like.

The sensor 24 also utilizes a clock so that the time of deposition of a sharp or group of sharps is determined and recorded and the time in service is measured.

Several suitable level sensors are available for use in one or more embodiments of the invention. As described above, sensor 24 is implemented by, for example, a beam emitter on one side of container 22 and a detector on the opposite side. The emitter and detector is positioned so that when the container is filled with sharps up to a predetermined level the beam is interrupted, thus triggering the notification.

Also, false signals arising from the detector responding to ambient light is minimized by modulating the beam according to a certain code and using detection electronics sensitive only to that code. A similar method is employed in infrared based television remote control systems. Various types of level sensors and sensing systems are also disclosed in Mallett et al., U.S. Patent Application Publication No. 20050065820.

In some embodiments, it is desirable to measure a fill level of waste within container 22 throughout the filling process. In some embodiments, such fill level sensing is performed by monitoring the weight of container 22, such as by using a load cell, balance, or other weight measurement device. In further embodiments, float systems are adapted for use in determining a level of a waste material. In some cases, it is also desirable to perform such fill level measurements without sensor 24 physically contacting container 22 or its contents.

In an alternative embodiment, a piezo transducer or the like are used to determine a volume of air remaining in container 22 by conducting a frequency sweep of the transducer to determine the resonance of the air in the container. Once the volume of air in container 22 is known, it is subtracted from the known total container volume to obtain the volume occupied by the container contents. In another alternative embodiment, a distance-measuring sensor (such as SONAR, RADAR or optical distance-measuring sensors) is located above and directed through the opening of container 22 in order to determine a "height" of the container contents. In yet another embodiment, sensor 24 determines whether container 22 includes any waste at all. Such a "waste presence" sensor is used in combination with a timer to determine a replacement schedule for a particular container based on a maximum acceptable dwell time for a particular waste item in a container. Still other embodiments use optical sensors to measure a fill level of container 22.

Another embodiment of a fill level sensing system comprises a light source and a light detector positioned on opposite sides of a disposable container. In alternative embodiments, the light detector need not be located immediately opposite the light source; for example, in some embodiments the detector is even located on a wall adjacent to the source. This sensor system generally operates on the principle that an "empty" container will permit more light to pass from the source, through container 22, and to sensor 24 than will a "full" container. This is simply due to the fact that the contents of the container will absorb and/or reflect a substantial portion of the light that enters container 22 from a light source.

In another embodiment, a light source is located at a "front" of the container and a detector is located at a "rear" of the container. In alternative embodiments, the positions of the light source and detector are reversed, or positioned at any other position around the container. In still further embodiments, multiple sources and/or detectors are used as desired.

As discussed above, the containers are typically made of a translucent material that allows at least some amount of light to pass through its walls. Optical sensing, for example, is particularly advantageous when used to measure a fill level of a container having translucent sidewalls. However, the skilled artisan will recognize that certain advantages of the embodiments described herein are advantageously applied to systems using containers having transparent sidewalls or containers with transparent windows in otherwise relatively opaque sidewalls.

The light source is any suitable source of light such as incandescent bulbs, white or colored LED's, or other sources. In some embodiments, the light source is located such that it is vertically centered on a desired "fill line" of container 22. The light source is laterally centered relative to the container, or have a width that is about as wide as the container. In still further embodiments, a plurality of light sources is used to illuminate a container from multiple points. The light detector is an array of photodetectors such as cadmium sulfide photodetectors or photodiodes. In some embodiments, it is also desirable to provide multiple rows of detectors.

For example, a middle row of detectors is positioned to lie just above the fill line of the container, and the lower row of detectors is positioned just below the fill line. The upper row of detectors is optionally located substantially above the fill line, and is used to calibrate the detector's middle and lower rows as will be described in further detail below. In some embodiments, rows are spaced apart (e.g., by about 1/2" to about 2 inches).

In operation, the individual photodetectors pick up light transmitted through the container and output corresponding signals to a processor. On one hand, the light intensity arriving at the detectors depends on the fill level of the container. In addition, a number of secondary factors also effect the light intensity reaching the detectors. These include the strength of the light source, the color and opacity of the container, the amount of ambient light, and other factors such as dust in the air. The light intensity at the top detector row is almost completely governed by these secondary factors, since it is located well above the fill line. By contrast, the light intensity arriving at the middle and lower detector rows will be effected more by the fill level of the container contents as the container becomes more full (e.g., as the fill level approaches the fill line).

When the container is empty and the overall light intensity is greatest, a baseline reading is recorded and calibration coefficients are generated for each of the detectors and detector rows. As the container fills, the received light reaching the detectors decreases slightly as material in the container blocks a portion of the diffused light transmitted through the container. During this phase, the top detector reading is used to compensate the readings of the middle and lower detector rows accordingly. When the container contents reaches the fill line, the bottom row of detectors will be blocked by the container contents, while the middle and upper detector rows remain unobstructed. This results in a substantial drop in the light intensity reaching the bottom row of detectors, and correspondingly, a substantial difference in signal strength between the middle and lower detector rows. When this signal difference reaches a pre-determined threshold level, the processor determines that the container is "full."

In some other embodiments, a parameter other than weight or filled volume is optionally used to determine when a container is "full." For example, in one embodiment, a sensor to detect radioactivity is used to determine the amount of radioisotope in a container or receptacle. The radioactivity sensor is used in connection with a fill sensor, or it is optionally used alone. Thus, in some embodiments, container 22 is emptied, discarded, or replaced based on a certain amount of radioactivity, rather than (or in addition to) the surface area, volume, weight, density and/or another parameter of the material in that container.

It is to be understood that the actual free surface of contents within container 22 is not necessarily planar. In such embodiments, the "fill plane" used by the processor and fill level sensing system is simply an average height of the waste material. In some embodiments, the items being deposited into container 22 are stacked unevenly or oddly oriented within container 22 so that its contents vary from a neat horizontal fill level. For example, some large items, such as syringes or other contaminated medical devices, stack oddly within container 22, thereby creating voids of unfilled space in a central portion of the container, above which waste items are stacked. Such variations in filling are suitable to lead to measurement errors. Thus, in some embodiments, a level sensing system is provided with error processing capabilities to account for variations in orientation and/or uneven loading of container 22.

As an example, the signals from a plurality of detectors are averaged to provide a consensus value for each respective detector row. This advantageously allows the processor to determine an average fill level in the event of an uneven fill surface. In an idealized case, for example, container 22 filled with a plurality of spherical particles through a hole in the top center of a regularly-shaped container will typically have a free surface in a shape of a cone with a peak at the center, and dropping off evenly in each direction. In such a case, the center detector of the lower row will typically receive a lower light intensity than the detectors on either side. Thus, by using the data from all of the detectors in a horizontal row, a processor calculates an approximate average fill level in order to prevent over-filling of container 22.

These or other error-processing techniques are also suitable to compensate for manufacturing defects in a container that might result in erroneous results. For example, if a plastic container wall comprises an air bubble or a dark spot in a region adjacent one or more of the detectors, these abnormalities are suitable to cause erroneous readings by those detectors. To compensate for this, a system gives less weight (or no weight at all) to signals from detectors that are out of a statistically expected range of variation from the remaining detectors. By taking an average signal across all detectors in various combinations and/or by assigning varying weights to individual detectors, a control algorithm teaches itself to recognize and adapt to such error-causing situations in order to obtain consistent readings.

In some embodiments, the functionality of a fill level sensing system employing a light source and a plurality of optical detectors is advantageously enhanced by containers with "frosted" or translucent walls. Another advantage of certain embodiments of a level sensing system as described herein is that such systems are polychromatic sensitive (i.e. configured to sense light of various colors with consistent accuracy). Thus, in addition to measuring a fill level of a container, the above-described sensors are configured to determine a color of a container (each container color being associated with a particular container type as discussed above). In some embodiments, these and other advantages are achieved through the use of cadmium sulfide photosensitive cells. In alternative embodiments, optical level sensors are constructed using other optical detectors, including other photoconductive cells, photo diodes, or other sensors capable of detecting light in the visible or infrared spectrum.

In some embodiments, each one of a plurality of fill-level sensors is controlled by a single processor in a waste sorting system. In one embodiment, a plurality of photodetector arrays are connected to a single multi-channel bus, and a plurality of light sources are controlled by a processor. In this embodiment, the processor illuminates a single container at a time. Thus, the detectors behind each of the "dark" containers would be at high impedance, and would therefore be out of the circuit.

In some embodiments, a fill level sensing system employing optical sources and detectors includes an additional photodetector that is generally configured to measure changes in "ambient" light within the system in order to appropriately adjust the readings from the detector arrays measuring fill level. An ambient light detector comprises a single optical detector, or a plurality of detectors in a circuit. In one such embodiment, an additional ambient light detector is provided within a waste sorting system in a location selected to measure any light entering the system from the exterior of the sorting system. For example, the ambient light detector is located adjacent a container-replacement door or any other portion of the system that is open to external light..

Various software algorithms are used by a level detector for use in a disposal system. As one example, when the system determines that a new container has been inserted, the level sensor establishes new baseline values for the detectors in order to define the "empty" state. The level sensing system then reads values of the detectors and inputs the detector values to an inference engine.

The inference engine uses for example a "fuzzy logic" method similar to the Sugeno method known in the art. In one embodiment, the inference engine uses a table of empirically-determined data to establish rule weights. The inference engine also uses multiple grouping of detectors in addition to individual detector levels to calculate a final fill level of container 22. In some embodiments, the empirically-determined lookup table is developed by performing various calibration experiments using an optical level sensing system to measure containers at known fill levels. In addition to any controlled experiments, the lookup table is supplemented by analysis of information it receives during use in measuring fill levels of new containers. For example, as optical anomalies are detected and accounted for, the software adapts to correct for them.

In order to avoid misleading readings during filling, the system is configured to determine when the detectors are at a steady state (e.g., when the movement of waste within the container drops below a threshold level). This is particularly helpful in embodiments in which a waste material is a liquid, and thus may continue moving for a period of time.

Once steady state is reached, the inference engine compares the values of the detector readings and ultimately derives a final fill value which is stored and/or output to a user-readable device such as a liquid crystal display. In alternative embodiments, an output of the system includes other visible, audible or tactile alerts, such as LEDs, buzzers, bells, vibrators, etc. In some embodiments, an output signal is used to notify the user that a particular container is ready to be emptied, discarded, replaced etc. In an alternative embodiment, an output signal is provided substantially continuously or at various intervals, so that the user determines or monitors the amount of material in a given container at any given time. For example, in some embodiments, the fill-level of container 22 is measured at regular intervals, such as every ten minutes, every hour, every two hours, every six hours, every 12 hours, or every 24 hours. In still further embodiments, the system comprises sensor 24 (such as an optical sensor) to determine when an item is deposited into container 22. Then a fill-level of the container 22 is measured after each item is deposited into it.

An alternative embodiment involves a video fill level sensing system. One embodiment employs a camera to continuously detect an intensity of light exiting container 22 from the source. In one embodiment, a light source is positioned to illuminate the container, and a curved mirror and pinhole video camera are located adjacent another side of the container. The system also includes a software-based processor and other electronic hardware. In the illustrated embodiment, the light source is located adjacent one vertical side of container 22 and the camera and mirror are positioned on the opposite side of the container. In alternative embodiments, the light source and camera/mirror assembly is located on adjacent sides of the container. Alternatively still, the light source is located above container 22 such that light is directed downward into the container, thereby allowing the waste to absorb as well as reflectively diffuse the light source onto the walls of the container.

In some embodiments, the camera is directed at the mirror to detect light emitted from the container and gathered by the mirror. The curved mirror provides a linearization of width by distorting the optics of the camera. In one embodiment, the camera is a pinhole camera, which is selected due to the depth of field this type of lens provides. In one embodiment, the curved mirror has a shape substantially similar to a shoehorn, e.g., it is curved about two perpendicular axes (e.g., longitudinal and transverse axes). Alternative mirror configurations are used as desired. The particular curvature of the mirror is determined empirically depending on the width of scanline needed and the height of the measured area (e.g., the height of the container wall). Variation in the curvature of the mirror along its length allows the scanline to be optimized in order to emphasize areas of higher interest and to de-emphasize lower interest areas. The mirror is for example convexly curved at the height of higher interest areas, and concavely curved to de-emphasize lower interest areas.

In some alternative embodiments, the light source includes bands of varying color or intensity along the height of the container in order to provide emphasis to portions of the container 22, or to provide "watermark" levels that are measured against. In some embodiments, the software is configured to interpret information received from the camera to learn about points of interest in order to further optimize a measurement algorithm. For example, rather than programming an algorithm to anticipate areas of higher or lower interest, the algorithm is configured to recognize variations in light intensity during calibration in order to detect such areas of higher or lower interest.

The processor and its support hardware provide the sampling of multiple luminance intensities along the wall of the container adjacent the mirror. The analog video signal is amplified and ground-referenced by the video amplifier. This amplified signal is scanned for a selected scanline to digitize for quantifying its luminance value. The amplified video is also applied to the Sync Separator module, which produces timing pulses for the scanline selector module. The processor receives the scanline data from the scanline selector, digitizer and sync separator. The video level sensor determines a current fill level of the waste in the container using a similar software method to that described above.

Many of the above embodiments of fill level sensor 24 were described with reference to a single disposable container 22. In some alternative embodiments, it is desirable to provide a single fill level detection system configured to selectively measure a fill level of any one of a plurality of containers 22. For example, in one embodiment, a light source is optionally provided on a first side of a plurality of containers, and a light detector is movable into a position opposite the light source of the containers. In one embodiment, this takes the form of a circular arrangement of containers in which a light detector is located at a center of a circular arrangement of containers. One or more light sources are positioned on an outer portion of the circular arrangement such that the light source and/or the light detector is capable of measuring a fill level of each one of the plurality of containers around the circle.

Some embodiments also include a weight scale (such as a load cell, pressure transducer, mechanical scale or other device) configured to weigh either a single spent sharp, container or individual segregated spent sharps. In one embodiment, the information from the scale are sent to a printer providing a means for printing a manifest for the container. Additionally, such information is combined with other information available to a clinician in order to determine a quantity of a sharp or substance that has been used or consumed.

A hospital optionally automates the dispensing of sharps. The automation is optionally embodied in a piece of equipment that a doctor or nurse accesses with a patient and clinician code and the correct amount of sharps may be dispensed. The automation provides pharmacists, nurses, doctors and administrators with information from a database on what sharps are dispensed and to which patient. These systems are typically indicate how many sharps were administered, but entering this information typically requires a clinician to return to the dispenser (which is inconvenient and, thus, not done regularly). This information is quite useful because it will demonstrate any inefficiencies or mistakes in administrating the sharps as well as point out any theft of sharps. In some embodiments, a sorting and disposal system is configured to track dispensing information because at the point of throwing the spent sharp away, they are automatically providing information to a central database.

Sensor systems based on weight are disclosed in U.S. Patent No. 4,961,533, U.S. Patent No. 6,418,841, and Published Patent Application 20020077875. Broadly speaking, the apparatus of 4,961,533 is useful for determining the volume contents of a plurality of containers, such as bottles containing alcoholic beverages, and comprises a transducer in operative contact with a surface for producing an output signal indicative of the weight of a container placed thereon and a sensor for receiving an output signal from a surface portion of the container.

Proximity sensors for detecting the level of sharps in a container are disclosed in Case, U.S. Patent No. 5,351,381. May et al., U.S. Patent Application Publication No. 20020108507, discloses details of proximity sensors, including some based on infra-red technology, used to detect the proximity of a human being to a waste container.

Level of sharps in the container is also optionally detected through the use of magnetic fields. For sharps made of ferromagnetic material such as iron-containing steel, the presence of sharps could distort a magnetic field already pervading the container. The distortions could be detected using any method of detecting magnetic fields, such as Hall-effect sensors.

As used herein, "empty" and "full" refer to relative amounts of waste, debris, or other matter, in container 22. For example, in certain embodiments, sensor 24 indicates that container 22 is ready to be emptied or discarded, not because it is completely full to its maximum capacity but because it has reached a desired point of fill or saturation. In some situations, it is desirous to empty or remove container 22 when anywhere from about 1% to about 100%, often from about 25% to about 100% of that container contains waste material. In other situations, it is desirable to remove a container when about 50% to about 95% of its volume is occupied by waste material.

Signal and data processing technologies embodying aspects of the present invention include fullness prediction algorithms and error processing to accommodate uneven stacking of sharps, perhaps making use of such techniques as fuzzy logic, as incorporated above. Self-troubleshooting and self-diagnostics are also implemented, in which a message is sent out alerting people to a problem or anticipating a problem. For example, Little, U.S. Patent No. 6,003,441, describes a system for self-monitoring of a waste disposal (compacting) system and Schomisch et al., U.S. Patent No. 5,967,028, suggests a method for predicting when a container is full or removed, even in the case when a sensor fails. In addition, Little et al., U.S. Patent No. 6,123,017, discusses a mathematical algorithm for predicting when a waste container will be full.

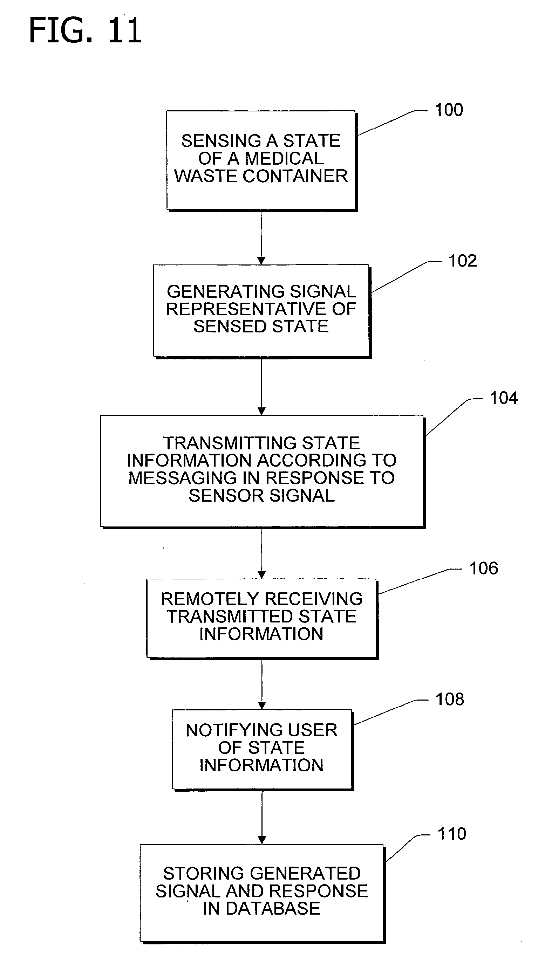

Referring now to FIG. 11, an exemplary flowchart illustrates operation of system 20. Beginning at 100, sensor 24 senses a state of medical waste container 22. In turn, sensor 24 generates a signal at 102, which is representative of the sensed state. Proceeding to 104, transmitter 32 (or RFID tag 92) transmits information representative of the sensed state according to a communications protocol. The remote receiver 34 (or RFID reader 94) receives the transmitted information at 106 for communication with notification device 36. In response to the information, notification device 36 notifies the user of the state information at 108. Moreover, one embodiment of the invention stores the sensor signal in the form of the message, for example, at 110. In addition, the response from the user is also stored.

It is to be understood that aspects of the invention are embodied by a general purpose computing device in the form of computer 58 or computer 66 (or one of the other exemplary devices 60, 62, 64). In one embodiment of the invention, a computer such as the computer 58 is suitable for use in the other figures illustrated and described herein. Computer 58 has one or more processors or processing units and a system memory. A system bus couples various system components including the system memory to the processors. The bus represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus also known as Mezzanine bus.

The computer 58 typically has at least some form of computer readable media. Computer readable media, which include both volatile and nonvolatile media, removable and non-removable media, is any available medium that is accessed by computer 58. By way of example and not limitation, computer readable media comprise computer storage media and communication media. Computer storage media include volatile and nonvolatile, removable and non-removable media implemented in any method or technology for storage of information such as computer readable instructions, data structures, program modules or other data. For example, computer storage media include RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium that is used to store the desired information and that is accessed by computer 58. Communication media typically embody computer readable instructions, data structures, program modules, or other data in a modulated data signal such as a carrier wave or other transport mechanism and include any information delivery media. Those skilled in the art are familiar with the modulated data signal, which has one or more of its characteristics set or changed in such a manner as to encode information in the signal. Wired media, such as a wired network or direct-wired connection, and wireless media, such as acoustic, RF, infrared, and other wireless media, are examples of communication media. Combinations of any of the above are also included within the scope of computer readable media.

A monitor or other type of display device is also connected to the system bus via an interface, such as a video interface. In addition to the monitor, or display, computers often include other peripheral output devices (not shown) such as a printer and speakers, which are connected through an output peripheral interface (not shown) for use in providing the notifications to the user.

The computer 58 operates in a networked environment using logical connections to one or more remote computers, such as a remote computer 60. The remote computer 60 is a personal computer, a server, a router, a network PC, a peer device or other common network node, and typically includes many or all of the elements described above relative to computer 58. The logical connections between computers include a local area network (LAN) and a wide area network (WAN), but also includes other networks. The LAN and/or WAN is a wired network, a wireless network, a combination thereof, and so on. Such networking environments are commonplace in offices, enterprise-wide computer networks, intranets, and global computer networks (e.g., the internet).

When used in a local area networking environment, computer 58 is connected to the LAN through a network interface or adapter. When used in a wide area networking environment, computer 58 typically includes a modem or other means for establishing communications over the WAN, such as the internet. The modem, which is internal or external, is connected to the system bus via a user input interface, or other appropriate mechanism. In a networked environment, program modules depicted relative to computer 58, or portions thereof, are stored in a remote memory storage device (not shown). The network connections shown are exemplary and other means of establishing a communications link between the computers are used.

Although described in connection with an exemplary computing system environment, including computer 58, one embodiment of the invention is operational with numerous other general purpose or special purpose computing system environments or configurations. The computing system environment is not intended to suggest any limitation as to the scope of use or functionality of embodiments of the invention. Moreover, the computing system environment should not be interpreted as having any dependency or requirement relating to any one or combination of components illustrated in the exemplary operating environment. Examples of well known computing systems, environments, and/or configurations that may be suitable for use with the embodiments of the invention include, but are not limited to, personal computers, server computers, hand-held or laptop devices, multiprocessor systems, microprocessor-based systems, set top boxes, programmable consumer electronics, mobile telephones, network PCs, minicomputers, mainframe computers, distributed computing environments that include any of the above systems or devices, and the like.

Embodiments of the invention may be described in the general context of computer-executable instructions, such as program modules, executed by one or more computers or other devices. Generally, program modules include, but are not limited to, routines, programs, objects, components, and data structures that perform particular tasks or implement particular abstract data types. Embodiments of the invention are also practiced in distributed computing environments where tasks are performed by remote processing devices that are linked through a communications network. In a distributed computing environment, program modules are located on both local and remote computer storage media including memory storage devices.

The order of execution or performance of the methods illustrated and described herein is not essential, unless otherwise specified. That is, it is contemplated by the inventors that elements of the methods are performed in any order, unless otherwise specified, and that the methods include more or less elements than those disclosed herein. For example, it is contemplated that executing or performing a particular element before, contemporaneously with, or after another element is within the scope of the invention.

When introducing elements of the present invention or the embodiments thereof, the articles "a", "an", "the", and "said" are intended to mean that there are one or more of the elements. The terms "comprising", "including", and "having" are intended to be inclusive and mean that there may be additional elements other than the listed elements.

In view of the above, it will be seen that the several objects of the invention are achieved and other advantageous results attained.

As various changes are realizable in the above constructions and methods without departing from the scope of embodiments of the invention, it is intended that all matter contained in the above description and shown in the accompanying drawings shall be interpreted as illustrative and not in a limiting sense.

## Claims

1. A medical device monitoring system comprising:
a sensor (24) associated with a medical device (70) for sensing a state of the medical device (70) and for generating a signal representative thereof; and
a notification device (36) wherein the notification device (36) is located remotely from the medical device (70) and receiving a message according to a messaging protocol (26), containing information representative of the sensed state of the medical device (70).

2. The medical device monitoring system according to claim 1, further comprising a transmitter (32) associated with the sensor (24) for transmitting the message according to the messaging protocol (26).

3. The medical device monitoring system according to claim 2, further comprising a receiver (34) associated with the notification device (36), wherein the receiver (34) and the transmitter (32) communicate with each other according to the messaging protocol (26).

4. The medical device monitoring system according to claim 3, wherein the receiver (34) and the transmitter (32) communicate with each other according to the messaging protocol (26) using one or more communications protocol.

5. The medical device monitoring system according to claim 4 wherein the communications protocol is a wireless communications protocol, an internet protocol, or a serial line communications protocol.

6. The medical device monitoring system according to claim 1 further comprising a communications network, wherein the sensor and the notification device comprise nodes on the network.

7. The medical device monitoring system according to any of the preceding claims, wherein the medical device (70) comprises one or more of the following: a medical waste container (22), a thermometer (72), an enteral feeding pump (78), and/or a compression apparatus for venous treatment.

8. The medical device monitoring system according to any of the preceding claims, wherein the notification device (36) comprises at least one of the following: a remote computing device (58), a telephone (64), a personal digital assistant (62), a wireless electronic mail device, and/or a pager.

9. A medical device comprising:
a medical waste container (22),
a sensor (24) associated with the waste container (22) for sensing a state of the waste container and for generating a signal representative thereof, and
a transmitter (32) responsive to the sensor signal for transmitting a message to a receiver (34) located remotely from the transmitter (32), wherein the message is transmitted according to a messaging protocol (26) and contains information representative of the sensed state of the waste container (22).

10. The medical device monitoring system according to claim 7 or the medical device according to claim 9 wherein the state of the waste container (22) is representative of one or more of the following: a level of medical waste items deposited in the waste container (22) relative to a capacity of the waste container (22); an uneven stacking condition within the waste container (22), a count of medical waste items deposited in the waste container (22), a time of last servicing of the waste container (22), a date of last servicing of the waste container (22), a location of the waste container and/or a service history of the waste container (22).

11. The medical device monitoring system according to any of claims 1 to 8, or the medical device according to claim 9, further comprising a radio frequency identification (RFID) tag (92) responsive to the sensor signal for storing information representative of the sensed state of the medical device (70).

12. The medical device monitoring system according to claim 11, or the medical device according to claim 9, further comprising an RFID reader (94) configured to read the information representative of the sensed state of the medical device (70) stored on the RFID tag (92).

13. The medical device monitoring system according to any of claims 1 to 8, or the medical device according to claim 9, wherein the sensor (24) comprises at least one of the following: an optical sensor, a weight sensor, an ultrasonic sensor, a radio frequency sensor, a capacitance sensor, an inductance sensor, a microwave sensor, a resonance frequency shift sensor, an imaging sensor, a proximity sensor, a solid sensor, a contact sensor, and/or a subsurface sensor.

14. The medical device monitoring system according to any of claims 1 to 8, or the medical device according to claim 9, wherein said messaging protocol (26) is selected from one or more of the following: TCP/IP, UDP/IP, MODBUS/TCP, HTTP, CANOpen, MODBUS, Profinet, and Profibus.

15. A method of monitoring one or more medical devices, said method comprising:
sensing (100) a state of a medical device (70);
communicating (104) a message containing information representative of the sensed state of the medical device (70), which is configured according to a messaging protocol (26) and transported using a communications protocol; and
remotely notifying (108) the sensed state of the medical device (70) in response to the communicated information.

16. The method according to claim 15, further comprising storing (110) the information representative of the sensed state of the medical device (70) in a database (68).

17. The method according to claim 16, further comprising analyzing the information representative of the sensed state of the medical device (70) stored in the database (68) and providing a notification to a user (80) in response thereto.

18. The method according to claim 16 or claim 17, further comprising analyzing the information representative of the sensed state of the medical device (70) stored in the database (68) and automatically ordering one or more replacement consumable items associated with the medical device (70) in response thereto.

19. The method according to any of claims 15 to 18, further comprising generating (102) a signal representative of the sensed state of the medical device (70) and, responsive to the sensor signal, transmitting (104) the message according to the communications protocol.

20. The method according to claim 19, further comprising receiving (106) the transmitted message representative of the sensed state of the medical device (70) according to any of the following communications protocols: a wireless communications protocol, an internet protocol, and/or a serial line communications protocol.

21. The method according to any of claims 15 to 20, wherein the medical device (70) is a waste container (22).

22. The method according to claim 21, wherein the state of the waste container (22) is representative of one or more of the following: a level of medical waste items deposited in the waste container (22) relative to a capacity of the waste container (22); an uneven stacking condition within the waste container (22), a count of medical waste items deposited in the waste container (22), a time of last servicing of the waste container (22), a date of last servicing of the waste container (22), a location of the waste container, and a service history of the waste container (22).

23. The method according to any of claims 15 to 22, further comprising storing information representative of the sensed state of the medical device (70) in a radio frequency identification (RFID) tag (92).

24. The method according to claim 23, further comprising reading the information representative of the sensed state of the medical device (70) stored on the RFID tag (92) and providing a notification to the user (80) in response thereto.

25. The method according to any of claims 15 to 24, further comprising communicating another message containing information representative of a state of one or more medical devices (70), wherein the message is configured according to the messaging protocol (26) and transported according to the communications protocol.

26. A computer-readable medium comprising:
a header (82) for permitting messaging according to a selected communications protocol,
a data section (86) containing information representative of a sensed state of a medical device (70), and
a message type (84) defining an operation to be performed on the data section (86), wherein the header, data section, and message type define a message configured according to a messaging protocol (26) for communication.
